(19)

Europäisches
Patentamt
European
Patent Office
Office européen
des brevets

(11)  **EP 4 353 739 A1**

(12)  **EUROPEAN PATENT APPLICATION**
published in accordance with Art. 153(4) EPC

(43) Date of publication:
**17.04.2024 Bulletin 2024/16**

(21) Application number: **22940501.4**

(22) Date of filing: **21.12.2022**

(51) International Patent Classification (IPC):
*C07K 14/31* (2006.01)  *C07K 19/00* (2006.01)
*C07K 16/00* (2006.01)  *C07K 1/22* (2006.01)
*C12N 15/31* (2006.01)  *C12N 15/62* (2006.01)
*C12N 15/70* (2006.01)  *C12N 1/21* (2006.01)
*G01N 33/68* (2006.01)  *B01D 15/38* (2006.01)
*C12R 1/19* (2006.01)

(86) International application number:
**PCT/CN2022/140536**

(87) International publication number:
**WO 2024/045430 (07.03.2024 Gazette 2024/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **01.09.2022  CN 202211064221**

(71) Applicant: **Sunresin New Materials Co., Ltd.
Xi'an, Shaanxi 710075 (CN)**

(72) Inventors:
• **LI, Yanjun
Xi'an
Shaanxi 710075 (CN)**
• **LI, Gang
Xi'an
Shaanxi 710075 (CN)**
• **GU, Tongnian
Xi'an
Shaanxi 710075 (CN)**

• **ZHANG, Jiantao
Xi'an
Shaanxi 710075 (CN)**
• **LIU, Long
Xi'an
Shaanxi 710075 (CN)**
• **LEI, Xiaoju
Xi'an
Shaanxi 710075 (CN)**
• **LIU, Qiong
Xi'an
Shaanxi 710075 (CN)**

(74) Representative: **J A Kemp LLP
80 Turnmill Street
London EC1M 5QU (GB)**

Remarks:
The complete document including Reference
Table(s) and the Sequence Listing(s) can be
downloaded from the EPO website

(54)  **POLYPEPTIDE, FUSED POLYMER PROTEIN AND USE THEREOF**

(57)  The present invention relates to the technical field of protein biological functions, in particular to a polypeptide, a fusion type multimeric protein and use thereof, the polypeptide is selected from: (1), a polypeptide having a substitution mutation in at least one position selected from the group consisting of positions 16, 25, 29, 49 and 58, as compared with a natural C structural domain of a protein A as shown in SEQ ID NO.1; wherein, the position 16 is subjected to a substitution mutation into leucine or valine; the position 25 is subjected to a substitution mutation into lysine, arginine, histidine or tryptophan; the position 29 is subjected to a substitution mutation into alanine, leucine or threonine; the position 49 is subjected to a substitution mutation into arginine or histidine; and the position 58 is subjected to a substitution mutation into glycine, isoleucine or alanine; or (2) a polypeptide having at least 80% of sequence identity to the polypeptide in (1) and retaining substitution mutation in at least one position selected from the group consisting of positions 16, 25, 29, 49 and 58; the polypeptide has high alkali tolerance and high loading capacity.

EP 4 353 739 A1

Figure 4

**Description**

**CROSS REFERENCE TO RELATED APPLICATIONS**

The present application claims priority to Chinese Patent Application No. 202211064221.6, entitled "POLYPEPTIDE, FUSION-TYPE MULTIMERIC PROTEIN AND USES THEREOF", and filed to the China National Intellectual Property Administration on September 1, 2022, the entire contents of which are incorporated herein by reference.

**TECHNICAL FIELD**

The present invention relates to the technical field of protein biological functional technology, in particular to a polypeptide, a fusion-type multimeric protein and uses thereof

**BACKGROUND**

As of February 2022, the FDA has approved a total of 109 antibody drugs, and the global antibody drug market has maintained a growth rate of over 10% for 8 consecutive years. In 2021, it exceeded $200 billion for the first time, with an increase of 16.5% compared to 2020. The separation of antibodies is crucial in the production of antibody drugs, and protein A affinity purification is a key step in antibody separation and purification. Currently, protein A fillers are sensitive to alkaline conditions, and the elution conditions are too strict. With the continuous expansion of antibody purification scale in the market, the requirements for their loading capacity and alkaline resistance performance are becoming higher and higher.

Staphylococcal Protein A (SPA), also known as Protein A, is a cell wall protein of *Staphylococcus aureus.* In 1983, the SPA gene was cloned and expressed in *Escherichia coli* by Duggleby et al. (Duggleby, C.J and Jones, SA: cloning and expression of the Staphylococcus aureus protein A gene in Escherichia coli. Nucl Acids Res. 11(1983)3065-3076; Lofdahl, S., Guss, B., et al; Gene for Staphylococcal protein A. Proc. Natl. Acid. Sci. USA 80(1983)697-701). SPA protein molecule is composed of five highly homologous structural domains E, D, A, B and C, and X structural domain, with a total of six structural domains. Because the five highly homologous structural domains E, D, A, B and C of SPA have the ability to bind to IgG and can bind to the Fc and Fab regions of most mammalian IgG (Jansson B, Uhlén M, Nygren PA. All individual domains of staphylococcal protein A show Fab binding. FEMS Immunol Med Microbiol. 1998 Jan; 20(1):69-78.). Therefore, SPA, as an affinity ligand for antibody purification coupled to solid-phase carrier, is widely used in the separation and purification of antibodies.

Due to the presence of impurities such as lipids, nucleic acids, proteins, polysaccharides, cell fragments, or contamination by viruses and bacteria in the sample during the antibody production process, these impurities may be non-specific adsorbed by protein A affinity fillers during the purification of antibodies, thereby contaminating the fillers. In addition, the protein A chromatography column also requires Clean In Place (CIP). In order to improve the service life of protein A filler and ensure column efficiency during its use, it is necessary to clean and regenerate the filler. The most widely used solution currently is to clean the chromatographic column in place with 0.1-1 M NaOH. However, natural protein A is intolerant to NaOH at concentrations higher than 0.1 M (Garshasb, Rigi, Samira, et al. A comprehensive review on staphylococcal protein A (SpA): Its production and applications. [J]. Biotechnology & Applied Biochemistry, 2019.), the development of genetic engineering has gradually improved the alkali resistance of recombinant protein A, but the high consumption and limited service life in industrial production of antibody drugs have led to increased production costs. Therefore, protein A fillers with higher loading capacity and higher alkali resistance are an inevitable trend pursued by antibody manufacturers and the development direction of filler production enterprises.

Antibodies are generally composed of Fab and Fc structural domains. The interaction between the Fab region of the antibody and SPA can seriously affect the elution pH value, leading to the need for lower pH values to achieve IgG dissociation and elution. The B structural domain mutant obtained by replacing glycine at position 29 of the B structural domain of the SPA molecule with alanine has almost no interaction with the Fab region. When asparagine at position 23 of the B structural domain is replaced by threonine, the alkali resistance of B structural domain mutant was significantly improved ( Amritkar V, Adat S, Tejwani V, et al. Engineering Staphylococcal Protein A for high-throughput affinity purification of monoclonal antibodies [J]. Biotechnology advances, 2020:107632.). However, the modified SPA molecules are still unable to meet the current demand for higher loading capacity and higher alkali resistance of SPA molecules.

**SUMMARY OF THE INVENTION**

Therefore, the technical problem to be solved in the present application is to provide a polypeptide, a fusion-type multimeric protein and uses thereof. The obtained fusion-type multimeric protein has higher loading capacity and higher alkali resistance.

Therefore, the present application provides the following technical solution:

According to one aspect of the present application, the present application provides a polypeptide, wherein the polypeptide is selected from:

(1) a polypeptide having a substitution mutation in at least one position selected from the group consisting of positions 16, 25, 29, 49 and 58, as compared with a natural C structural domain of a protein A as shown in SEQ ID NO.1; wherein,

the position 16 is subjected to a substitution mutation into leucine or valine;
the position 25 is subjected to a substitution mutation into lysine, arginine, histidine or tryptophan;
the position 29 is subjected to a substitution mutation into alanine, leucine or threonine;
the position 49 is subjected to a substitution mutation into arginine or histidine; and
the position 58 is subjected to a substitution mutation into glycine, isoleucine or alanine; or

(2) a polypeptide having at least 80% of sequence identity to the polypeptide in (1) and retaining substitution mutation in at least one position selected from the group consisting of positions 16, 25, 29, 49 and 58.

Optionally, the position 16 is subjected to a substitution mutation into leucine; and/or

the position 25 is subjected to a substitution mutation into lysine; and/or
the position 29 is subjected to a substitution mutation into alanine; and/or
the position 49 is subjected to a substitution mutation into arginine; and/or
the position 58 is subjected to a substitution mutation into glycine.

Optionally, the polypeptide has the substitution mutation in position 58, and has the substitution mutation in at least one position selected from the group consisting of positions 16, 25, 29 and 49; or

the polypeptide has the substitution mutation in position 49, and has the substitution mutation in at least one position selected from the group consisting of positions 16, 25, 29 and 58; or
the polypeptide has the substitution mutation in position 29, and has the substitution mutation in at least one position selected from the group consisting of positions 16, 25, 49 and 58; or
the polypeptide has the substitution mutation in position 25, and has the substitution mutation in at least one position selected from the group consisting of positions 16, 29, 49 and 58; or
the polypeptide has the substitution mutation in position 16, and has the substitution mutation in at least one position selected from the group consisting of positions 25, 29, 49 and 58; or
optionally, the polypeptide has substitution mutations at positions 16, 25, 29, 49 and 58; or
optionally, the polypeptide has substitution mutations at positions 16, 25, 49 and 58; or
optionally, the polypeptide has substitution mutations at positions 49 and 58; or
optionally, the polypeptide has substitution mutations at positions 29, 49 and 58; or
optionally, the polypeptide has substitution mutations at positions 16, 25 and 29; or
optionally, the polypeptide has substitution mutations at positions 16 and 25; or
optionally, the polypeptide has a substitution mutation at position 49; or
optionally, the polypeptide has a substitution mutation at position 25.

According to another aspect of the present application, the present application provides a fusion-type multimeric protein, wherein the fusion-type multimeric protein comprises the fusion-type multimeric protein formed by fusion of the polypeptide.

The fusion-type multimeric protein further comprises polypeptide B and/or polypeptide Zmb; wherein the polypeptide B satisfies any of the following conditions:

a. having an amino acid sequence as shown in SEQ ID NO.2; and
b. having an amino acid sequence of at least 80% sequence identity to the amino acid sequence as shown in SEQ ID NO.2 in a;

the polypeptide Zmb satisfies any of the following conditions:

c. inserting an amino acid sequence ML in at least one position of the amino acid sequence shown in SEQ ID NO.2 in a, wherein the amino acid sequence of the amino acid sequence ML is shown in SEQ ID NO.3; and

EP 4 353 739 A1

d. inserting an amino acid sequence ML in at least one position of amino acid sequence described in b; optionally, the amino acid sequence of the polypeptide Zmb is shown in SEQ ID NO.4.

The fusion-type multimeric protein, wherein the polypeptide is designated polypeptide Cm; the fusion-type multimeric protein comprises at least one polypeptide Cm, and/or at least one polypeptide Zmb;

optionally, the fusion-type multimeric protein comprises 1 to 6 polypeptides Cm, and/or 1 to 4 polypeptides Zmb; optionally, the fusion-type multimeric protein comprises 4 polypeptides Cm and/or 2 polypeptides Zmb.

The fusion-type multimeric protein, further comprises at least one functional polypeptide FLD, wherein the at least one functional polypeptide FLD satisfies any of the following conditions:

A. having an amino acid sequence shown in SEQ ID NO.5; and
B. having an amino acid sequence of at least 80% sequence identity to the amino acid sequence as shown in SEQ ID NO.5 in A;

optionally, the functional polypeptide FLD is located between the polypeptide Cm and the polypeptide Zmb; optionally, the fusion-type multimeric protein comprises 4 polypeptides Cm, 1 functional polypeptide FLD and 2 polypeptides Zmb sequentially from N-terminal to C-terminal.

According to another aspect of the present application, the present application provides a biomaterial, wherein the biomaterial comprises any of the following:

1) a nucleic acid molecule encoding the polypeptide or the fusion-type multimeric protein; optionally, the nucleic acid molecule is DNA or RNA;
2) an expression cassette, a recombinant vector, a recombinant microorganism or a transgenic cell line expressing the polypeptide or the fusion-type multimeric protein;
3) an expression cassette, a recombinant vector, a recombinant microorganism or a transgenic cell line containing the nucleic acid molecule described in 1);
4) a recombinant vector, recombinant microorganism or transgenic cell line containing the expression cassette described in 2) or 3); and
5) a host cell containing the recombinant vector described in 2) or 3) or 4);

optionally, the recombinant vector is constructed from the *Escherichia coli* expression vector pET-30a(+); optionally, the host cell is *Escherichia coli,* and optionally, the *Escherichia coli* is *Escherichia coli* BL21(DE3).

Use of the polypeptide or the fusion-type multimeric protein in antibody detection, separation or purification.
According to another aspect of the present application, the present application provides an affinity chromatography medium, wherein the affinity chromatography medium comprises the fusion-type multimeric protein and an affinity chromatography medium vector;
optionally, the affinity chromatography medium vector includes agarose gel, dextran, cellulose, high molecular polymer with hydroxyl groups or silica gel.
The technical solution of the present application has the following advantages:

1. The present application provides a polypeptide, wherein the polypeptide is selected from: (1), a polypeptide having a substitution mutation in at least one position selected from the group consisting of positions 16, 25, 29, 49 and 58, as compared with a natural C structural domain of a protein A as shown in SEQ ID NO.1; wherein, the position 16 is subjected to a substitution mutation into leucine or valine; the position 25 is subjected to a substitution mutation into lysine, arginine, histidine or tryptophan; the position 29 is subjected to a substitution mutation into alanine, leucine or threonine; the position 49 is subjected to a substitution mutation into arginine or histidine; and the position 58 is subjected to a substitution mutation into glycine, isoleucine or alanine; or (2) a polypeptide having at least 80% of sequence identity to the polypeptide in (1) and retaining substitution mutation in at least one position selected from the group consisting of positions 16, 25, 29, 49 and 58. The polypeptide used has high alkali resistance and high loading capacity, resulting in higher loading capacity and alkali resistance of the fusion-type multimeric protein prepared using this polypeptide.
2. The fusion-type multimeric protein provided in the present application comprises the fusion-type multimeric protein formed by fusion of the polypeptide, and has higher loading capacity and higher alkali resistance.
3. The fusion-type multimeric protein provided in the present application also comprises polypeptide Zmb, and the amino acid sequence of the polypeptide Zmb is shown in SEQ ID NO.4. The experimental results show that when the polypeptide Zmb structural domain is used to prepare filler for purifying antibodies, the pH value of the elution buffer is increased from 3.3 to 4.3.

4. The fusion-type multimeric protein provided in the present application also comprises at least one functional polypeptide FLD. The insertion of the FLD reduces the steric hindrance effect of the three-dimensional structure of the fusion type polymer protein, enabling it to recombine to IgG, thereby improving the overall loading capacity of the fusion-type multimeric protein.

5. The affinity chromatography medium provided in the present application comprises the fusion-type multimeric protein and an affinity chromatography medium vector. The fusion-type multimeric protein can be coupled with a chromatography medium vector to form an affinity chromatography medium for antibody detection, separation, or purification.

**BRIEF DESCRIPTION OF THE DRAWINGS**

To describe the technical solutions in specific implementations of the present invention or the prior art more clearly, the following briefly introduces the accompanying drawings required for describing the specific implementations or the prior art. Apparently, the accompanying drawings in the following description show some implementations of the present invention, and a person of ordinary skill in the art may still derive other drawings from these accompanying drawings without creative efforts.

Figure 1 is the SDS-PAGE gel electrophoresis result of BL21-pET30a-CmZmb bacterial fluid in Example 5 of the present application; in the figure, lane 1 is before lactose induction; lane 2 is lactose induced; lane 3 is the lysate supernatant 1; lane 4 is the lysate supernatant 2; lane 5 is the lysate supernatant 3; lane 6 is eluent 1; and lane 7 is eluent 2; lane 8 is a protein marker (provided by Shaanxi Proanti Biotechnology Development Co., Ltd., Article No. : 10225-1);

Figure 2 is the results of purified protein of BL21-pET30a-CmZmb bacterial fluid in Example 5 of the present application; in the figure, lane 1 is the lysate supernatant 2; lane 2 is a flowing liquid; lane 3 is eluent; lane 4 is 4% B solution for eluting impurities; lane 5 is 8% B solution for eluting impurities; lane 6 is 40% B solution for eluting target protein; and lane 7 is a 100% B solution for eluting target protein.

Figure 3 shows the dynamic loading capacity test results of the fusion-type multimeric protein in Experiment Example 1 of the present application;

Figure 4 shows the dynamic loading capacity test results of the alkali resistance of the fusion-type multimeric protein in Experiment Example 2 of the present application.

**DETAILED DESCRIPTION**

The following examples are provided in order to further understand the present invention better, are not limited to the best implementation mode, and do not limit the content and protection scope of the present invention, anyone under the inspiration of the present invention or use the present invention Any product identical or similar to the present invention obtained by combining features of other prior art falls within the protection scope of the present invention.

If no specific experimental steps or conditions are indicated in the examples, it can be carried out according to the operation or conditions of the conventional experimental steps described in the literature in this field. The reagents or instruments used, whose manufacturers are not indicated, are all commercially available conventional reagent products.

The coding gene of polypeptides and fusion-type multimeric protein in the following examples were synthesized by Nanjing GenScript Biotechnology Co., Ltd.

In the present invention, amino acid residues can be the following abbreviations: lysine (K), glycine (G), alanine (A), arginine (R), glutamic acid (E), isoleucine (I), leucine (L).

Example 1: Polypeptide Cm

1. In order to explore the alkali resistance solution of the polypeptide Cm, a series of polypeptide Cm are designed in this example, and the details of the polypeptide are as follows:

Polypeptide C (natural): the amino acid sequence is shown in SEQ ID NO.1.
Polypeptide Cm (K58G): compared with the natural C structural domain of protein A shown in SEQ ID NO.1, the polypeptide only has a substitution mutation of lysine (K) at position 58 to glycine (G).

Polypeptide Cm (K49R): compared with the natural C structural domain of protein A shown in SEQ ID NO.1, the polypeptide only has a substitution mutation of lysine (K) at position 49 to arginine (R).

Polypeptide Cm (G29A): compared with the natural C structural domain of protein A shown in SEQ ID NO.1, the polypeptide only has a substitution mutation of glycine (G) at position 29 to alanine (A).

Polypeptide Cm (E25K): compared with the natural C structural domain of protein A shown in SEQ ID NO.1, the polypeptide only has a substitution mutation of glutamic acid (E) at position 25 to lysine (K).

Polypeptide Cm (I16L): compared with the natural C structural domain of protein A shown in SEQ ID NO.1, the polypeptide only has a substitution mutation of isoleucine (I) at position 16 to leucine (L).

Polypeptide Cm (K58G, K49R, G29A, E25K, I16L): compared with the natural C structural domain of protein A shown in SEQ ID NO. 1, the polypeptide has:

> a substitution mutation of lysine (K) at position 58 to glycine (G);
> a substitution mutation of lysine (K) at position 49 to arginine (R);
> a substitution mutation of glycine (G) at position 29 to alanine (A);
> a substitution mutation of glutamic acid (E) at position 25 to lysine (K); and
> a substitution mutation of isoleucine (I) at position 16 to leucine (L).

Polypeptide Cm (K58G, K49R, E25K, I16L): compared with the natural C structural domain of protein A shown in SEQ ID NO.1, the polypeptide has:

> a substitution mutation of lysine (K) at position 58 to glycine (G);
> a substitution mutation of lysine (K) at position 49 to arginine (R);
> a substitution mutation of glutamic acid (E) at position 25 to lysine (K); and
> a substitution mutation of isoleucine (I) at position 16 to leucine (L).

Polypeptide Cm (K58G, K49R): compared with the natural C structural domain of protein A shown in SEQ ID NO.1, the polypeptide has:

> a substitution mutation of lysine (K) at position 58 to glycine (G); and
> a substitution mutation of lysine (K) at position 49 to arginine (R).

Polypeptide Cm (K58G, K49R, G29A): compared with the natural C structural domain of protein A shown in SEQ ID NO.1, the polypeptide has:

> a substitution mutation of lysine (K) at position 58 to glycine (G);
> a substitution mutation of lysine (K) at position 49 to arginine (R); and
> a substitution mutation of glycine (G) at position 29 to alanine (A).

Polypeptide Cm (G29A, E25K, I16L): compared with the natural C structural domain of protein A shown in SEQ ID NO.1, the polypeptide has:

> a substitution mutation of glycine (G) at position 29 to alanine (A);
> a substitution mutation of glutamic acid (E) at position 25 to lysine (K); and
> a substitution mutation of isoleucine (I) at position 16 to leucine (L).

Polypeptide Cm (E25K, I16L): compared with the natural C structural domain of protein A shown in SEQ ID NO.1, the polypeptide has:

> a substitution mutation of glutamic acid (E) at position 25 to lysine (K); and
> a substitution mutation of isoleucine (I) at position 16 to leucine (L).

Further, the substitution mutation at position 16 involved in the above polypeptide Cm can also be a substitution mutation into valine;

the substitution mutation at position 25 can also be a substitution mutation into arginine or histidine or tryptophan;
the substitution mutation at position 29 is a substitution mutation into leucine or threonine;
the substitution mutation at position 49 is a substitution mutation into histidine; and

the substitution mutation at position 58 is a substitution mutation into isoleucine or alanine.

Example 2

In this example, the polypeptide Zmb is designed, and the polypeptide Zmb is as follows:

Polypeptide B: having an amino acid sequence as shown in SEQ ID NO.2;
Polypeptide Zmb: inserting an amino acid sequence ML between position 20 and position 21 of the amino acid sequence shown in SEQ ID NO.2, wherein the amino acid sequence of the amino acid sequence ML is shown in SEQ ID NO.3; that is the amino acid sequence of the polypeptide Zmb is shown in SEQ ID NO.4.

Example 3

In this example, a functional polypeptide FLD is designed, and the functional polypeptide FLD has an amino acid sequence as shown in SEQ ID NO.5.

Example 4: Fusion-type multimeric protein

In this example, a series of fusion-type multimeric proteins were designed using the polypeptides of Examples 1-3, as follows:

CC: an amino acid sequence fused with six polypeptide C (natural) sequentially from N-terminal to C-terminal, that is, the arrangement is: C-C-C-C-C-C.
CmCm (K58G): an amino acid sequence fused with six polypeptides Cm (K58G) sequentially from the N-terminal to the C-terminal.
CmCm (K49R): an amino acid sequence fused with six polypeptides Cm (K49R) sequentially from the N-terminal to the C-terminal.
CmCm (G29A): an amino acid sequence fused with six polypeptide Cm (G29A) sequentially from the N-terminal to the C-terminal.
CmCm (E25K): an amino acid sequence fused with six polypeptides Cm (E25K) sequentially from the N-terminal to the C-terminal.
CmCm (I16L): an amino acid sequence fused with six polypeptides Cm (I16L) sequentially from the N-terminal to the C-terminal.
CmCm (K58G, K49R, G29A, E25K, I16L): an amino acid sequence fused with six polypeptide Cm (K58G, K49R, G29A, E25K, I16L) sequentially from the N-terminal to the C-terminal.
CmCm (K58G, K49R, E25K, I16L): an amino acid sequence fused with six polypeptide Cm (K58G, K49R, E25K, I16L) sequentially from N-terminal to C-terminal.
CmCm (K58G, K49R): an amino acid sequence fused with six polypeptides Cm (K58G, K49R) sequentially from the N-terminal to the C-terminal.
CmCm (K58G, K49R, G29A): an amino acid sequence fused with six polypeptide Cm (K58G, K49R, G29A) sequentially from the N-terminal to the C-terminal.
CmCm (G29A, E25K, I16L): an amino acid sequence fused with six polypeptide Cm (G29A, E25K, I16L) sequentially from the N-terminal to the C-terminal.
CmCm (E25K, I16L): an amino acid sequence fused with six polypeptides Cm (E25K, I16L) sequentially from the N-terminal to the C-terminal.
CDB: an amino acid sequence fused from N-terminal to C-terminal by 4 polypeptides C (natural), 1 functional polypeptide FLD, and 2 polypeptides B. The arrangement is: C-C-C-C-FLD-B-B.
CB: from the N-terminal to the C-terminal, the amino acid sequence is fused with 4 polypeptides C (natural) and 2 polypeptides B, and the arrangement is: C-C-C-C-B-B.
CmZmb: from the N-terminal to the C-terminal, the amino acid sequence is fused with 4 polypeptides Cm, 1 functional polypeptide FLD, and 2 polypeptides Zmb. The arrangement is: Cm-Cm-Cm-Cm-FLD-Zmb-Zmb. Its amino acid sequence is shown in SEQ ID NO.6, and its gene sequence is shown in SEQ ID NO.7.

Further, the substitution mutation at position 16 involved in the above polypeptide in the above-mentioned fusion-type multimeric protein can also be a substitution mutation into valine;

the substitution mutation at position 25 can also be a substitution mutation into arginine or histidine or tryptophan;
the substitution mutation at position 29 is a substitution mutation into leucine or threonine;

the substitution mutation at position 49 is a substitution mutation into histidine; and
the substitution mutation at position 58 is a substitution mutation into isoleucine or alanine.

Example 5: Preparation of fusion-type multimeric protein

In this example, the fusion-type multimeric protein designed in Example 4 is prepared, and the preparation process is as follows:

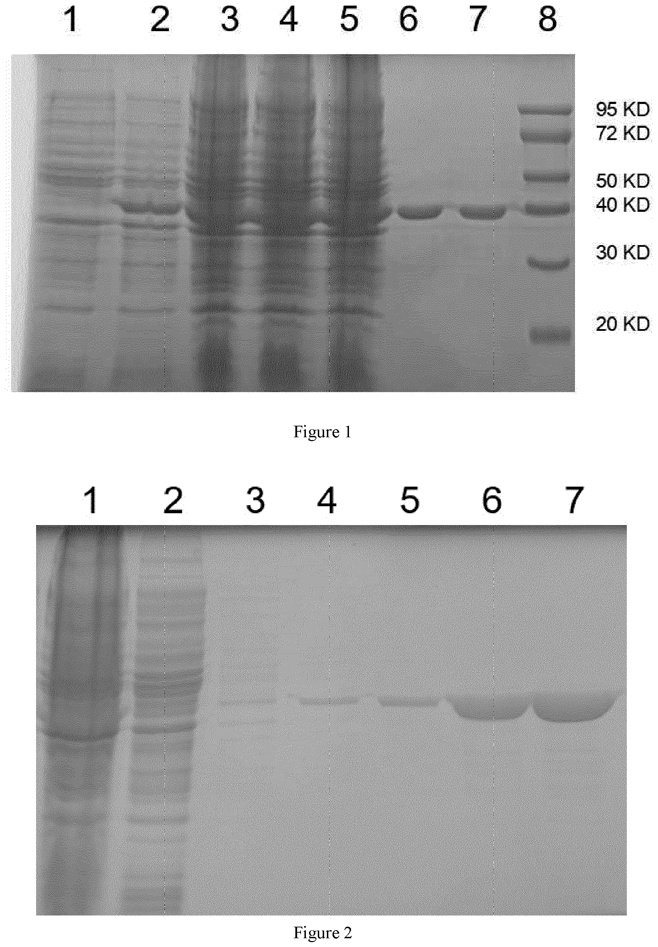

(1) Construction of recombinant vector
The coding gene the fusion-type multimeric protein and the expression vector pET-30a(+) were double enzyme digested by NdeI (purchased from Shenggong Biotechnology (Shanghai) Co., Ltd., B600120) and HindIII (purchased from Shenggong Biotechnology (Shanghai) Co., Ltd., B600184), and the corresponding fragments were recovered and connected (DNA gel recovery kit, purchased from Shanghai Biyuntian Biotechnology Co., Ltd., D0056). The connected product was mixed with the top 10 receptive cells of *Escherichia coli,* and was placed on ice for 30 min, heat-shocked in the water bath at 42 °C for 90 s, placed on ice for transformation 3 min, 100μl room temperature LB liquid medium was added, and cultured in a shaker at 37 °C at 220 rpm for 60 min, the bacterial fluid was mixed well and applied to kanamycin (purchased From Shengong Bioengineering (Shanghai) Co., Ltd., A506636) resistant plate, the plate was inverted, cultured overnight at 37 °C, and the transformant with kanamycin resistance was screened, thereby screening to obtain a recombinant vector expressing the fusion-type multimeric protein. For example, the recombinant vector expressing the fusion-type multimeric protein CmZmb obtained by screening is named pET-30a-CmZmb. Similarly, the recombinant vectors for other fusion-type multimeric proteins were named.
(2) Construction of recombinant bacteria
Fresh BL21 (DE3) bacterial fluid was ice-bathed for 30min, 4°C, centrifuged at 1000g for 10min, and the precipitate was resuspended with 0.1mol/L $MgCl_2$-$CaCl_2$ (80mmol $MgCl_2$ and 80mmol $CaCl_2$, in which MgClz and CaClz were purchased from Sinopharm Chemical Reagent Co., Ltd., analytical pure, and the rest chemical reagents have the same purity), 4°C, centrifuged at 1000g for 10min, then resuspended in 0.1mol/L CaClz solution to prepare BL21 (DE3) competent cells, the recombinant vector (such as pET-30a-CmZmb) screened in step (1) into competent cells of *Escherichia coli* BL21 (DE3), the transformant was screened on the LB plate containing kanamycin, and it was verified to be a recombinant transformant by sequencing verify, named BL21-pET30a-CmZmb. Similarly, the recombinant transformants for other fusion-type multimeric proteins were named.
(3) Fermentation of recombinant bacteria
The recombinant transformants (such as BL21-pET30a-CmZmb) with correct sequencing were inoculated in LB medium with an inoculum volume of 0.5% (volume percentage), cultured overnight at 37 °C until the $OD_{600}$ reached 0.8, and lactose with a final concentration of 10g/L was added for inducing, bacteria was collected by centrifugation after 3-5 hours;
10 μl of 5× loading buffer (provided by Shaanxi Proanti Biotechnology Development Co., Ltd., Article No.: 10137-1) was added to 40 μl of bacterial fluid (pre lactose induced bacterial fluid and post lactose induced bacterial fluid, respectively) and boiled for 5 min, sample loading 20 μL to carry out SDS-PAGE gel electrophoresis, and the results were shown in Figure 1. It can be seen that a new protein band appears at the position of 44kD in the lactose-induced BL21-pET30a-CmZmb bacterial liquid (see Figure 1 lane 2), the BL21-pET30a-CmZmb bacterial fluid without lactose induction does not appear this band or this band is lighter in color (see Figure 1 lane 1). This demonstrated that the fusion-type multimeric protein CmZmb was induced to express in BL21-pET30a-CmZmb, similarly, other fusion-type multimeric protein in Example 4 were also successfully induced to express.
(4) Purification

Analytical pure sodium chloride was added to a solution containing 20 mM PB (prepared using 5.8018 g/L of disodium hydrogen phosphate dodecahydrate aqueous solution and 0.5928 g/L of disodium hydrogen phosphate dihydrate aqueous solution, with standard buffer preparation method) to a concentration of 11.688 g/L. The pH was adjusted to 7.4 using an acid-base aqueous solution to obtain buffer solution A. The bacterial liquid collected in the above step (3) was suspended with the buffer A liquid according to the weight ratio of 1:10, the lysis time was 20min, 30min, 40min respectively, ultrasonically crushed, and the supernatant was collected after centrifugation at room temperature to obtain the supernatant of the lysate supernatants 1, 2 and 3. Simultaneously, the lysate supernatant 2 was subjected to nickel column affinity purification (according to the following lysate supernatant 2 (lysis time 30min) to carry out the nickel column affinity purification operation until elution with 40% B solution, and the eluate was collected and divided into two parts, i.e. eluent 1 and eluent 2). SDS-PAGE electrophoresis detection shows that the results are shown in lanes 3-7 in Figure 1 (the samples using three lysis times show no difference in results, see lanes 3-5 in Figure 1, and the lysate supernatant 1 in lane 3 corresponds to a lysis time of 20 min, the lysate supernatant 2 in lane 4 corresponds to a lysis time of 30 min, and the lysate supernatant 3 in lane 5 corresponds to a lysis time of 40 min. The results of eluent 1 and eluent 2 show no significant difference, see lanes 6-7 in Figure 1), most of the fusion-type multimeric protein was expressed in the periplasm of *E. coli,* and the expression amount in the precipitate was less.

The lysate supernatant 2 (lysis time 30min) was subjected to nickel column affinity purification, the specific purification steps: the flow rate of the purifier (ÄKTA, Purifier) was adjusted to 3ml/min, the column was equilibrated with buffer A to make A280nm (detection wavelength), A231nm (detection wavelength) and A215nm (detection wavelength) baselines are leveled by about 10CV, and the baseline was flat; the sample was loaded through the A pump (lysate supernatant 60-120ml); the chromatographic column was washed with buffer A and flattened to the baseline by about 10CV, the impurities was washed with 8% (percentage by volume) of B solution (B solution is buffer A with a final concentration of 17.02 g/L imidazole, which was purchased from Sinopod Chemical Reagents Co., LTD.), and flatten to the baseline about 10CV; 40% (volume percentage) B solution was used for eluting the target protein, and the baseline was about 10CV; B solution (100% (volume percentage)) was used for eluting about 3CV, until the baseline was flat (the absorption of protein at A280nm was very small, the absorption of protein at A215nm was high, but imidazole has a great influence on A215nm, when collecting protein, it is necessary to repeatedly confirm the collection range, when the concentration was low, and the collection should start at the peak of A215nm or A231nm when the concentration is low and when the concentration is high, it can be collected according to the peak of A280nm); the chromatographic column and the chromatographic system were washed with buffer A until the baseline flat is about 10CV The purified recombinant proteins with characteristic peaks, such as the fusion-type multimeric protein CmZmb with a purity of more than 80% (the results are shown in the swimming lane in Figure 2) were collected, and similarly, the purity of other fusion-type multimeric proteins can also reach more than 80%.

Example 6: Preparation of affinity chromatography medium

This example provides the preparation of affinity chromatography medium, as follows:

The fusion-type multimeric protein obtained in Example 5 was used to prepare affinity chromatography media respectively, and the specific steps were as follows: 2-30 mL (15ml was selected in this example) cyanogen bromide was added to 100 g of Sepharose 4FF (SUNRESIN NEW MATERIALS CO.LTD.), reacted at -10°C to 10°C (0°C was selected in this example) for 15 to 30min (22min was selected in this example), 2 to 50mL (25ml was selected in this example) triethylamine was added, and reacted at -10°C to 10°C (0°C was selected in this example) for 15 to 60min (35min was selected in this example), washed with acetone; 0.2 to 2g (1g was selected in this example) fusion-type multimeric protein was dissolved with pure water, was added to above-mentioned agarose gel 4FF, pH was adjusted to 7 to 11 (pH=8 was selected in this example), reacted at 0-20°C (10°C was selected in this example) overnight, 0.1-10% (volume percentage, 5% in this example) ethanolamine solution of pH 7 to 11 was added, reacted overnight at 0°C to 40°C (20°C was selected in this example) to obtain an affinity chromatography medium. The prepared affinity chromatography medium is stored in 20% (volume percent) ethanol solution.

Experimental example 1: Dynamic capacity test of fusion-type multimeric protein filler

The ÄKTA pure chromatography system programming was used to carry out the experiment, the chromatography column medium was the affinity chromatography medium prepared in Example 6, 1.1ml affinity chromatography medium was mixed well with 20% (volume percentage) 2ml of ethanol aqueous solution, constant flow pump (purchased from Shanghai Qingpu Huxi Instrument Factory) was used to loaded the chromatography column medium into the chromatographic column at a flow rate of 8rpm/min, the detection wavelength was set to 280 nm, the pre-column pressure was 0.3MPa, the chromatographic column was connected to the system, and the flow rate was set to 1.0ml /min, rinse 10CV with mobile phase B1, equilibrate 15CV with mobile phase A1; load sample (2mg/ml IgG, purchased from Beijing Suo Laibao Technology Co., Ltd., SP031) flow rate 0.2ml/min, load sample to the absorb value at 280nm is 90mAu; constant elution mobile phase was set to 100% (volume percentage) mobile phase B1, flow rate was set to 1.0ml/min, eluted to baseline flat, the eluent was collected; Finally, 10 ml was regenerated using mobile phase A1 at a flow rate of 1.0 ml/min. The absorption value is dynamically detected and monitored from the start to the end of the program

Mobile phase A1: 0.15M NaCl containing 20mm PB, pH 7.4;

Mobile phase B1: 0.1M citric acid-sodium citrate buffer, pH3.3;

$$\text{Dynamic load} = (V_A \times C_0)/V_C(\text{mg/ml gel})$$

$C_0$ is the concentration of the target protein in the sample; $V_A$ is the total volume of the sample loaded when the concentration of the target sample in the breakthrough curve reaches 5% C0; Vc is the total column bed volume (see Liu Ying, Zeng Jiancheng, Yu Miao, et al. A method for determining the dynamic loading capacity of an affinity chromatography filler: CN 114280208 A [P].)

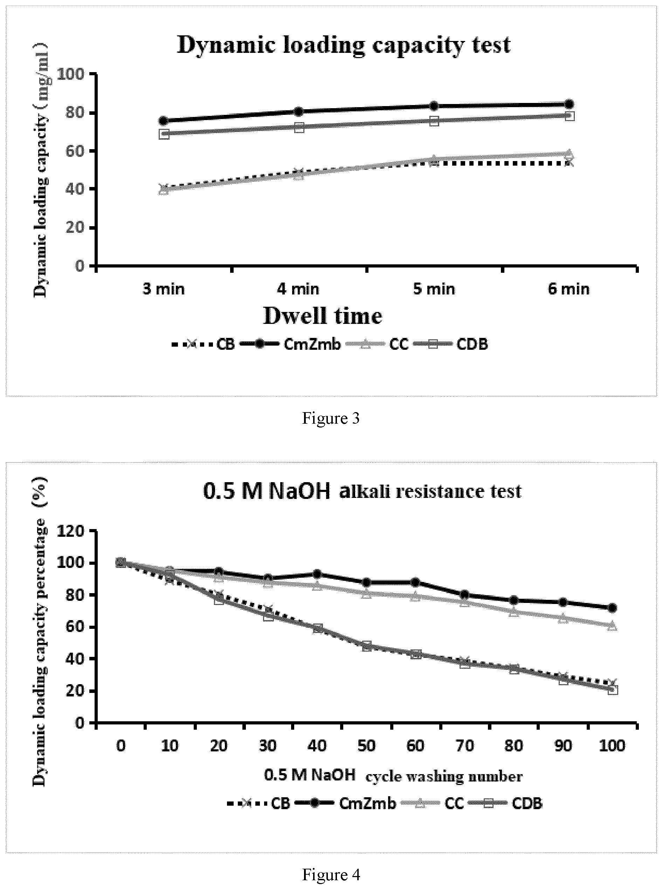

The dynamic loading capacity of fillers prepared with CmZmb, CC, CDB, and CB (peptide C in CC, CDB, and CB are all polypeptide C (natural)) was tested using the above method. The results are shown in Figure 3, and the dynamic loading capacity of fillers prepared with CmZmb is higher than that of fillers prepared with CC, CDB, and CB.

Implement according to the above-mentioned method, when elution buffer pH4.3 (pH of mobile phase B1 is 4.3), the elution efficiency of CmZmb, CC, CDB and CB were respectively 86.6%, 56.8%, 87.9% and 62.3%. The affinity chromatography medium prepared by CmZmb has a relatively higher efficiency in eluting the target protein with a pH 4.3 buffer compared to other affinity chromatography media.

Experimental example 2: Alkali resistance test of fusion-type multimeric protein filler

The ÄKTA pure chromatography system programming was used to carry out the experiment, the chromatography column medium was the affinity chromatography medium prepared in Example 6, 1.1ml affinity chromatography medium was mixed well with 20% (volume percentage) 2ml of ethanol aqueous solution, constant flow pump (purchased from Shanghai Qingpu Huxi Instrument Factory) was used to loaded the chromatography column medium into the chromatographic column at a flow rate of 8rpm/min, the detection wavelength was set to 280 nm, the pre-column pressure was 0.3MPa, the chromatographic column was connected to the system, and buffer A2 was used to equilibrate at a flow rate of 0.5mL/min for 10min, wash off the ethanol in the column, wash the column with 0.5M NaOH at a flow rate of 0.2mL/min for 15min, then wash the column with buffer A2 at a flow rate of 0.5mL/min for 10min, the above step was repeated 100 cycles, and the dynamic load was determined after every 10 cycles (see Experimental Example 1 for the calculation method of the dynamic loading capacity). Buffer A2: 0.15M NaCl containing 20mM PB, pH7.4.

The alkali resistance test results of the fusion-type multimeric protein prepared only from polypeptide C (native) or polypeptide Cm were shown in the table below, partly shown in Figure 4.

Table 1. Alkali resistance test results of fillers prepared from fusion-type multimeric protein

| Fillers prepared from fusion-type multimeric protein | Initial dynamic loading capacity (mg/m l) | Dynamic loading capacity after 100 cycles (mg/ml) | Dynamic loading capacity percentage after 100 cycles (%) |
|---|---|---|---|
| CC | 58.26 | 35.27 | 60.54 |
| CmCm (K58G) | 41.29 | 24.18 | 58.56 |
| CmCm (K49R) | 57.69 | 36.91 | 63.98 |
| CmCm (G29A) | 45.69 | 29.99 | 65.64 |
| CmCm (E25K) | 42.98 | 27.07 | 62.98 |
| CmCm (I16L) | 48.47 | 28.58 | 58.96 |
| CmCm (K58G, K49R, G29A, E25K, I16L) | 56.02 | 41.31 | 73.74 |
| CmCm (K58G, K49R, E25K, I16L) | 55.68 | 37.13 | 66.69 |
| CmCm (K58G, K49R) | 46.78 | 29.16 | 62.34 |
| CmCm (K58G, K49R, G29A) | 55.94 | 38.18 | 68.25 |
| CmCm (G29A, E25K, I16L) | 49.68 | 33.18 | 66.78 |
| CmCm (E25K, I16L) | 48.56 | 29.73 | 61.23 |

2. The alkali resistance test results of the fillers prepared by CmZmb, CC, CDB and CB were shown in Figure 4. It can be seen from the figure that the percentage of dynamic loading in the alkali resistance test of the fillers prepared by CmZmb is higher than that of CC, CDB and CB.

Obviously, the foregoing embodiments are merely examples for clear description, rather than a limitation to implementations. For a person of ordinary skill in the art, other changes or variations in different forms may also be made based on the foregoing description. All implementations cannot and do not need to be exhaustively listed herein. Obvious changes or variations that are derived there from still fall within the protection scope of the invention of the present invention.

**Claims**

1. A polypeptide, wherein the polypeptide is selected from:

    (1), a polypeptide having a substitution mutation in at least one position selected from the group consisting of positions 16, 25, 29, 49 and 58, as compared with a natural C structural domain of a protein A as shown in SEQ ID NO.1; wherein,

       the position 16 is subjected to a substitution mutation into leucine or valine;
       the position 25 is subjected to a substitution mutation into lysine, arginine, histidine or tryptophan;
       the position 29 is subjected to a substitution mutation into alanine, leucine or threonine;
       the position 49 is subjected to a substitution mutation into arginine or histidine; and
       the position 58 is subjected to a substitution mutation into glycine, isoleucine or alanine; or

    (2) a polypeptide having at least 80% of sequence identity to the polypeptide in (1) and retaining substitution mutation in at least one position selected from the group consisting of positions 16, 25, 29, 49 and 58.

2. The polypeptide of claim 1, wherein the position 16 is substituted and mutated to leucine; and/or

    the position 25 is subjected to a substitution mutation into lysine; and/or
    the position 29 is subjected to a substitution mutation into alanine; and/or
    the position 49 is subjected to a substitution mutation into arginine; and/or
    the position 58 is subjected to a substitution mutation into glycine.

3. The polypeptide of claim 1 or 2, wherein the polypeptide has the substitution mutation in position 58, and has the substitution mutation in at least one position selected from the group consisting of positions 16, 25, 29 and 49; or

    the polypeptide has the substitution mutation in position 49, and has the substitution mutation in at least one position selected from the group consisting of positions 16, 25, 29 and 58; or
    the polypeptide has the substitution mutation in position 29, and has the substitution mutation in at least one position selected from the group consisting of positions 16, 25, 49 and 58; or
    the polypeptide has the substitution mutation in position 25, and has the substitution mutation in at least one position selected from the group consisting of positions 16, 29, 49 and 58; or
    the polypeptide has the substitution mutation in position 16, and has the substitution mutation in at least one position selected from the group consisting of positions 25, 29, 49 and 58; or
    optionally, the polypeptide has substitution mutations at positions 16, 25, 29, 49 and 58; or
    optionally, the polypeptide has substitution mutations at positions 16, 25, 49 and 58; or
    optionally, the polypeptide has substitution mutations at positions 49 and 58; or
    optionally, the polypeptide has substitution mutations at positions 29, 49 and 58; or
    optionally, the polypeptide has substitution mutations at positions 16, 25 and 29; or
    optionally, the polypeptide has substitution mutations at positions 16 and 25; or
    optionally, the polypeptide has a substitution mutation at position 49; or
    optionally, the polypeptide has a substitution mutation at position 25.

4. A fusion-type multimeric protein, wherein the fusion-type multimeric protein comprises the fusion-type multimeric protein formed by fusion of the polypeptide according to any one of claims 1-3.

5. The fusion-type multimeric protein of claim 4, wherein the fusion-type multimeric protein further comprises polypeptide B and/or polypeptide Zmb; wherein

    the polypeptide B satisfies any of the following conditions:

       a. having an amino acid sequence as shown in SEQ ID NO.2; and
       b. having an amino acid sequence of at least 80% sequence identity to the amino acid sequence as shown in SEQ ID NO.2 in a;

the polypeptide Zmb satisfies any of the following conditions:

 c. inserting an amino acid sequence ML in at least one position of the amino acid sequence shown in SEQ ID NO.2 in a, wherein the amino acid sequence of the amino acid sequence ML is shown in SEQ ID NO.3; and
 d. inserting an amino acid sequence ML in at least one position of amino acid sequence described in b;

optionally, the amino acid sequence of the polypeptide Zmb is shown in SEQ ID NO.4.

6. The fusion-type multimeric protein according to claim 4 or 5, wherein the polypeptide according to any one of claims 1-4 is designated polypeptide Cm; the fusion-type multimeric protein comprises at least one polypeptide Cm, and/or at least one polypeptide Zmb;

 optionally, the fusion-type multimeric protein comprises 1 to 6 polypeptides Cm, and/or 1 to 4 polypeptides Zmb;
 optionally, the fusion-type multimeric protein comprises 4 polypeptides Cm and/or 2 polypeptides Zmb.

7. The fusion-type multimeric protein according to any one of claims 4-6, further comprising at least one functional polypeptide FLD, wherein the at least one functional polypeptide FLD satisfies any of the following conditions:

 A. having an amino acid sequence shown in SEQ ID NO.5; and
 B. having an amino acid sequence of at least 80% sequence identity to the amino acid sequence as shown in SEQ ID NO.5 in A;
 optionally, the functional polypeptide FLD is located between the polypeptide Cm and the polypeptide Zmb;
 optionally, the fusion-type multimeric protein comprises 4 polypeptides Cm, 1 functional polypeptide FLD and 2 polypeptides Zmb sequentially from N-terminal to C-terminal.

8. A biomaterial, wherein the biomaterial comprises any of the following:

 1) a nucleic acid molecule encoding the polypeptide according to any one of claims 1-3 or the fusion-type multimeric protein according to any one of claims 4-7; optionally, the nucleic acid molecule is DNA or RNA;
 2) an expression cassette, a recombinant vector, a recombinant microorganism or a transgenic cell line expressing the polypeptide according to any one of claims 1-3 or the fusion-type multimeric protein according to any one of claims 4-7;
 3) an expression cassette, a recombinant vector, a recombinant microorganism or a transgenic cell line containing the nucleic acid molecule described in 1);
 4) a recombinant vector, recombinant microorganism or transgenic cell line containing the expression cassette described in 2) or 3); and
 5) a host cell containing the recombinant vector described in 2) or 3) or 4);

  optionally, the recombinant vector is constructed from the *Escherichia coli* expression vector pET-30a(+);
  optionally, the host cell is *Escherichia coli,* and optionally, the *Escherichia coli* is *Escherichia coli* BL21(DE3).

9. Use of the polypeptide according to any one of claims 1-3 or the fusion-type multimeric protein according to any one of claims 4-7 in antibody detection, separation or purification.

10. An affinity chromatography medium, wherein the affinity chromatography medium comprises the fusion-type multimeric protein according to any one of claims 4-7 and affinity chromatography medium vector;
optionally, the affinity chromatography medium vector includes but is not limited to agarose gel, dextran, cellulose, high molecular polymer with hydroxyl groups or silica gel.

Figure 1

Figure 2

Figure 3

Figure 4

## INTERNATIONAL SEARCH REPORT

International application No.

**PCT/CN2022/140536**

### A. CLASSIFICATION OF SUBJECT MATTER

C07K14/31(2006.01)i; C07K19/00(2006.01)i; C07K16/00(2006.01)i; C07K1/22(2006.01)i; C12N15/31(2006.01)i; C12N15/62(2006.01)i; C12N15/70(2006.01)i; C12N1/21(2006.01)i; G01N33/68(2006.01)i; B01D15/38(2006.01)i; C12R1/19(2006.01)i

According to International Patent Classification (IPC) or to both national classification and IPC

### B. FIELDS SEARCHED

Minimum documentation searched (classification system followed by classification symbols)

IPC: C07K, C12N, G01N, B01D, C12R

Documentation searched other than minimum documentation to the extent that such documents are included in the fields searched

Electronic data base consulted during the international search (name of data base and, where practicable, search terms used)

Databases: CNTXT, WPABSC, WPABS, DWPI, VEN, ENTXTC, ENTXT, CNKI, 万方, WANFANG, 百度学术, Baidu Scholar, ISI Web of Science, PubMed, Elsevier Science, GenBank, EMBL, 中国专利生物序列检索系统, China Patent Biological Sequence Search System, STN; search terms: 多肽, polypeptide, 蛋白A, 金黄色葡萄球菌蛋白A, staphylococal protein A, SPA, C结构域, C-domain, 修饰, 突变, modified, mutant, 16, I16L, I16V, 亮氨酸, L, Leu, leucine, 缬氨酸, Val, V, valine, 取代, 抗体, antibody, 纯化, 亲和层析, purification, 载量, loading capacity, 耐碱, 稳定, alkali tolerance, alkaline stability, 对SEQ ID NOs: 1-6进行序列检索, search for SEQ ID NOs: 1-6.

### C. DOCUMENTS CONSIDERED TO BE RELEVANT

| Category* | Citation of document, with indication, where appropriate, of the relevant passages | Relevant to claim No. |
|---|---|---|
| A | Vinod Amritkar et al. "Engineering Staphylococcal Protein A for High-throughput Affinity Purification of Monoclonal Antibodies" *Biotechnology Advances*, Vol. vol. 44, 22 September 2020 (2020-09-22), see section 3, figure 4, table 1 | 1-10 (in part) |
| A | CN 114149495 A (BESTCHROM (SHANGHAI) BIOSCIENCES LTD.; BESTCHROM (ZHEJIANG) BIOSCIENCES LTD.) 08 March 2022 (2022-03-08) see claims 1-23 | 1-10 (in part) |
| A | CN 112639099 A (JSR CORPORATION; JSR MICRO INC.; JSR MICRO N.V.) 09 April 2021 (2021-04-09) see embodiments 1-5, test examples 1-3, tables 1-3 and 7 | 1-10 (in part) |
| A | CN 105377880 A (GE HEALTHCARE BIO-SCIENCES AB) 02 March 2016 (2016-03-02) see claims 1-34 | 1-10 (in part) |

☑ Further documents are listed in the continuation of Box C.　　☑ See patent family annex.

| | | |
|---|---|---|
| * | Special categories of cited documents: | |
| "A" | document defining the general state of the art which is not considered to be of particular relevance | |
| "D" | document cited by the applicant in the international application | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **18 May 2023** | **28 May 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2022/140536** |

**C.     DOCUMENTS CONSIDERED TO BE RELEVANT**

| Category* | Citation of document, with indication, where appropriate, of the relevant passages | Relevant to claim No. |
|---|---|---|
| A | CN 102895960 A (EMD MILLIPORE CORPORATION) 30 January 2013 (2013-01-30)<br>      see claims 1-20 | 1-10 (in part) |
| A | CN 102365361 A (KANEKA CORP.) 29 February 2012 (2012-02-29)<br>      see claims 1-25 | 1-10 (in part) |
| A | US 2017327534 A1 (GE HEALTHCARE BIOPROCESS R&D AB) 16 November 2017<br>(2017-11-16)<br>      see claims 1-20 | 1-10 (in part) |
| A | CN 103443120 A (KANEKA CORP.) 11 December 2013 (2013-12-11)<br>      see claims 1-3 | 1-10 (in part) |

Form PCT/ISA/210 (second sheet) (July 2022)

**INTERNATIONAL SEARCH REPORT**

| International application No. |
|---|
| **PCT/CN2022/140536** |

| Box No. I    Nucleotide and/or amino acid sequence(s) (Continuation of item 1.c of the first sheet) |
|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2022/140536** |

| Box No. III    Observations where unity of invention is lacking (Continuation of item 3 of first sheet) |
| --- |

This International Searching Authority found multiple inventions in this international application, as follows:

Invention 1: Claims 1-10 (all in part) relate to a polypeptide, which, compared to native C domain of protein A as shown in SEQ ID NO. 1, has a leucine or valine substitution mutation at the 16th site and further has a substitution mutation at at least one site selected from the 25th site, the 29th site, the 49th site and the 58th site; and a related polypeptide having at least 80% homology, fusion multimeric protein, biological material, use, and affinity chromatography medium.

Invention 2: Claims 1-10 (all in part) relate to a polypeptide, which, compared to native C domain of protein A as shown in SEQ ID NO. 1, has a lysine or arginine or histidine or tryptophan substitution mutation at the 25th site and further has a substitution mutation at at least one site selected from the 16th site, the 29th site, the 49th site and the 58th site; and a related polypeptide having at least 80% homology, fusion multimeric protein, biological material, use, and affinity chromatography medium.

Invention 3: Claims 1-10 (all in part) relate to a polypeptide, which, compared to native C domain of protein A as shown in SEQ ID NO. 1, has an alanine or leucine or threonine substitution mutation at the 29th site and further has a substitution mutation at at least one site selected from the 16th site, the 25th site, the 49th site and the 58th site; and a related polypeptide having at least 80% homology, fusion multimeric protein, biological material, use, and affinity chromatography medium.

Invention 4: Claims 1-10 (all in part) relate to a polypeptide, which, compared to native C domain of protein A as shown in SEQ ID NO. 1, has an arginine or histidine substitution mutation at the 49th site and further has a substitution mutation at at least one site selected from the 16th site, the 25th site, the 29th site and the 58th site; and a related polypeptide having at least 80% homology, fusion multimeric protein, biological material, use, and affinity chromatography medium.

Invention 5: Claims 1-10 (all in part) relate to a polypeptide, which, compared to native C domain of protein A as shown in SEQ ID NO. 1, has a glycine or isoleucine or alanine substitution mutation at the 58th site and further has a substitution mutation at at least one site selected from the 16th site, the 25th site, the 29th site and the 49th site; and a related polypeptide having at least 80% homology, fusion multimeric protein, biological material, use, and affinity chromatography medium.

The same or corresponding technical feature among inventions 1-5 is: a polypeptide having an amino acid substitution mutation in native C domain of protein A. However, the feature is disclosed in the prior art, for example, D1 (CN 112639099 A, 09 April 2021 (09.04.2021)) (see embodiments 1-5, and test examples 1-3). Therefore, the five inventions do not have a same or corresponding special technical feature that defines a contribution which the inventions make over the prior art, and therefore do not comply with the requirement of unity of invention and do not comply with PCT Rule 13.1.

**INTERNATIONAL SEARCH REPORT**

International application No.

**PCT/CN2022/140536**

**Box No. III        Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-10 (in part)**

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
Information on patent family members

International application No.

**PCT/CN2022/140536**

| Patent document cited in search report | | | Publication date (day/month/year) | Patent family member(s) | | | Publication date (day/month/year) |
|---|---|---|---|---|---|---|---|
| CN | 114149495 | A | 08 March 2022 | None | | | |
| CN | 112639099 | A | 09 April 2021 | EP | 3842536 | A1 | 30 June 2021 |
| | | | | EP | 3842536 | A4 | 18 January 2023 |
| | | | | WO | 2020040307 | A1 | 27 February 2020 |
| | | | | KR | 20210049802 | A | 06 May 2021 |
| | | | | TW | 202024328 | A | 01 July 2020 |
| | | | | JPWO | 2020040307 | A1 | 26 August 2021 |
| | | | | US | 2021179671 | A1 | 17 June 2021 |
| CN | 105377880 | A | 02 March 2016 | US | 2016159855 | A1 | 09 June 2016 |
| | | | | US | 9896486 | B2 | 20 February 2018 |
| | | | | WO | 2015005859 | A1 | 15 January 2015 |
| | | | | JP | 2016523959 | A | 12 August 2016 |
| | | | | JP | 6544808 | B2 | 17 July 2019 |
| | | | | US | 2017320922 | A1 | 09 November 2017 |
| | | | | US | 10112980 | B2 | 30 October 2018 |
| | | | | EP | 3019519 | A1 | 18 May 2016 |
| | | | | EP | 3019519 | A4 | 08 March 2017 |
| | | | | EP | 3019519 | B1 | 03 April 2019 |
| | | | | US | 2016159857 | A1 | 09 June 2016 |
| | | | | US | 10308690 | B2 | 04 June 2019 |
| | | | | EP | 3019520 | A1 | 18 May 2016 |
| | | | | EP | 3019520 | A4 | 08 March 2017 |
| | | | | EP | 3019520 | B1 | 11 December 2019 |
| | | | | WO | 2015005862 | A1 | 15 January 2015 |
| | | | | JP | 2016525100 | A | 22 August 2016 |
| | | | | JP | 6544809 | B2 | 17 July 2019 |
| | | | | US | 2016207966 | A1 | 21 July 2016 |
| | | | | US | 9663558 | B2 | 30 May 2017 |
| | | | | JP | 2019165747 | A | 03 October 2019 |
| | | | | JP | 6862008 | B2 | 21 April 2021 |
| | | | | US | 2018244729 | A1 | 30 August 2018 |
| | | | | US | 11136359 | B2 | 05 October 2021 |
| CN | 102895960 | A | 30 January 2013 | JP | 2017019792 | A | 26 January 2017 |
| | | | | JP | 6189493 | B2 | 30 August 2017 |
| | | | | JP | 2012254981 | A | 27 December 2012 |
| | | | | JP | 5756428 | B2 | 29 July 2015 |
| | | | | IL | 229774 | A | 31 August 2017 |
| | | | | ES | 2671722 | T3 | 08 June 2018 |
| | | | | TW | 201305191 | A | 01 February 2013 |
| | | | | TWI | 596108 | B | 21 August 2017 |
| | | | | US | 2015252085 | A1 | 10 September 2015 |
| | | | | US | 9234010 | B2 | 12 January 2016 |
| | | | | EP | 2532672 | A2 | 12 December 2012 |
| | | | | EP | 2532672 | A3 | 03 April 2013 |
| | | | | EP | 2532672 | B1 | 04 April 2018 |
| | | | | TW | 201716427 | A | 16 May 2017 |
| | | | | TWI | 638828 | B | 21 October 2018 |
| | | | | SG | 10201604559 | TA | 28 July 2016 |
| | | | | US | 2016168194 | A1 | 16 June 2016 |
| | | | | US | 9376474 | B1 | 28 June 2016 |

Form PCT/ISA/210 (patent family annex) (July 2022)

**INTERNATIONAL SEARCH REPORT**
Information on patent family members

International application No.

**PCT/CN2022/140536**

| Patent document cited in search report | | | Publication date (day/month/year) | Patent family member(s) | | | Publication date (day/month/year) |
|---|---|---|---|---|---|---|---|
| | | | | KR | 20180035201 | A | 05 April 2018 |
| | | | | KR | 101928697 | B1 | 12 December 2018 |
| | | | | JP | 2018021038 | A | 08 February 2018 |
| | | | | JP | 6586131 | B2 | 02 October 2019 |
| | | | | RU | 2644680 | C1 | 13 February 2018 |
| | | | | WO | 2013109302 | A2 | 25 July 2013 |
| | | | | WO | 2013109302 | A3 | 17 October 2013 |
| | | | | TW | 201542586 | A | 16 November 2015 |
| | | | | TWI | 570134 | B | 11 February 2017 |
| | | | | RU | 2013154493 | A | 20 July 2015 |
| | | | | RU | 2595430 | C2 | 27 August 2016 |
| | | | | AU | 2012366229 | A1 | 07 November 2013 |
| | | | | AU | 2012366229 | B2 | 17 November 2016 |
| | | | | AU | 2012366229 | C1 | 09 March 2017 |
| | | | | AU | 2017200616 | A1 | 23 February 2017 |
| | | | | AU | 2017200616 | B2 | 01 November 2018 |
| | | | | ES | 2710204 | T3 | 23 April 2019 |
| | | | | IL | 253992 | A0 | 31 October 2017 |
| | | | | IL | 253992 | B | 31 January 2019 |
| | | | | EP | 3053931 | A2 | 10 August 2016 |
| | | | | EP | 3053931 | A3 | 26 October 2016 |
| | | | | EP | 3053931 | B1 | 02 January 2019 |
| | | | | JP | 2014193892 | A | 09 October 2014 |
| | | | | JP | 6018120 | B2 | 02 November 2016 |
| | | | | US | 2014296434 | A1 | 02 October 2014 |
| | | | | US | 8895706 | B2 | 25 November 2014 |
| | | | | SG | 186552 | A1 | 30 January 2013 |
| | | | | DK | 2532672 | T3 | 14 June 2018 |
| | | | | DK | 3053931 | T3 | 04 March 2019 |
| | | | | KR | 20140044348 | A | 14 April 2014 |
| | | | | KR | 101844107 | B1 | 30 March 2018 |
| | | | | KR | 20120137259 | A | 20 December 2012 |
| | | | | KR | 101464040 | B1 | 20 November 2014 |
| | | | | SG | 10201604554 | WA | 28 July 2016 |
| | | | | AU | 2019200368 | A1 | 07 February 2019 |
| | | | | AU | 2019200368 | B2 | 11 March 2021 |
| | | | | US | 2013046056 | A1 | 21 February 2013 |
| | | | | US | 8754196 | B2 | 17 June 2014 |
| | | | | US | 2015080558 | A1 | 19 March 2015 |
| | | | | US | 9018305 | B2 | 28 April 2015 |
| CN | 102365361 | A | 29 February 2012 | EP | 2412809 | A1 | 01 February 2012 |
| | | | | EP | 2412809 | A4 | 29 May 2013 |
| | | | | EP | 2412809 | B1 | 09 August 2017 |
| | | | | US | 2012208234 | A1 | 16 August 2012 |
| | | | | US | 9403883 | B2 | 02 August 2016 |
| | | | | JP | 2016025872 | A | 12 February 2016 |
| | | | | JP | 6184463 | B2 | 23 August 2017 |
| | | | | JPWO | 2010110288 | A1 | 27 September 2012 |
| | | | | WO | 2010110288 | A1 | 30 September 2010 |
| US | 2017327534 | A1 | 16 November 2017 | US | 10654887 | B2 | 19 May 2020 |

Form PCT/ISA/210 (patent family annex) (July 2022)

**INTERNATIONAL SEARCH REPORT**
Information on patent family members

International application No.

**PCT/CN2022/140536**

| Patent document cited in search report | | | Publication date (day/month/year) | Patent family member(s) | | | Publication date (day/month/year) |
|---|---|---|---|---|---|---|---|
| CN | 103443120 | A | 11 December 2013 | JPWO | 2012133342 | A1 | 28 July 2014 |
| | | | | JP | 5933526 | B2 | 08 June 2016 |
| | | | | EP | 2690108 | A1 | 29 January 2014 |
| | | | | EP | 2690108 | A4 | 31 December 2014 |
| | | | | EP | 2690108 | B1 | 25 July 2018 |
| | | | | CA | 2830990 | A1 | 04 October 2012 |
| | | | | KR | 20140007394 | A | 17 January 2014 |
| | | | | SG | 193950 | A1 | 29 November 2013 |
| | | | | US | 2014100356 | A1 | 10 April 2014 |
| | | | | US | 9284354 | B2 | 15 March 2016 |
| | | | | WO | 2012133342 | A1 | 04 October 2012 |
| | | | | AU | 2012233980 | A1 | 17 October 2013 |
| | | | | AU | 2012233980 | B2 | 13 October 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211064221
- CN 114280208 A

**Non-patent literature cited in the description**

- **DUGGLEBY, C.J ; JONES, SA.** cloning and expression of the Staphylococcus aureus protein A gene in Escherichia coli. *Nucl Acids Res.,* 1983, vol. 11, 3065-3076
- **LOFDAHL, S ; GUSS, B. et al.** Gene for Staphylococcal protein. *A. Proc. Natl. Acid. Sci. USA,* 1983, vol. 80, 697-701
- **JANSSON B ; UHLÉN M ; NYGREN PA.** All individual domains of staphylococcal protein A show Fab binding. *FEMS Immunol Med Microbiol,* January 1998, vol. 20 (1), 69-78
- **GARSHASB ; RIGI ; SAMIRA et al.** A comprehensive review on staphylococcal protein A (SpA): Its production and applications. [J]. *Biotechnology & Applied Biochemistry,* 2019
- **AMRITKAR V ; ADAT S ; TEJWANI V et al.** Engineering Staphylococcal Protein A for high-throughput affinity purification of monoclonal antibodies [J]. *Biotechnology advances,* 2020, 107632